# EUROPÄISCHE PATENTANMELDUNG

(11) **EP 2 189 114 A1**
(43) Veröffentlichungstag der Anmeldung: **26.05.2010**
(21) Anmeldenummer: 09154868.5
(22) Anmeldetag: 11.03.2009
(51) Int. Cl.: A61B 6/00, A61B 6/04

(54) **Vorrichtung zur Fixierung der weiblichen Brust für die diagnostische Bildgebung und Intervention**

(30) Priorität: 22.11.2008 DE 102008058587
(71) Anmelder: MIR Medical Imaging Research Holding GmbH, 91096 Möhrendorf (DE)
(72) Erfinder: Tita, Ralf, 69434, Hirschhorn/Neckar (DE)
(74) Vertreter: Lohr, Georg

(57) **Zusammenfassung**

Eine Vorrichtung zur Fixierung einer Brust (31) einer Patientin in einem medizinischen Untersuchungsgerät umfasst eine elastische ringförmige Fixierung (110,114), sowie ein daran befestigtes Netz (113), welche die Brust (31) an ihrem Ansatz an der Brustwand eng umschließen. Dabei wird auf die Brust (31) eine Kraft in einer Richtung von der Patientin weg ausgeübt wird, um die Brust (31) zu komprimieren und von der Brustwand abzuheben.

## Beschreibung

### Technisches Gebiet

Die Erfindung betrifft eine Vorrichtung zur Fixierung der weiblichen Brust, anwendbar bei der diagnostischen Bildaufnahme und der interventionellen Therapie. Eine derartige Vorrichtung dient zur Fixierung der Brust in einer für die bildgebende, diagnostische Untersuchung vorteilhaften und reproduzierbaren Position. Diese Fixierung kann darüber hinaus auch für eine Fixierung bei einer Gewebeprobenentnahme (Biopsie) oder einer anderen Art der Diagnose eines verdächtigen Gewebes oder einer Therapie genutzt werden.

### Stand der Technik

Zur Untersuchung der weiblichen Brust sind verschiedene Geräte, wie Röntgengeräte oder auch CT-Scanner bekannt. Ein solcher CT-Scanner ist beispielsweise in der US 2006/0094950 A1 offenbart. Unter einer Liege, auf der eine zu untersuchende Patientin liegt, befindet sich eine Röntgenvorrichtung mit einer rotierenden Gantry, welche eine Röntgenröhre und einen Detektor aufweist. Die zu untersuchende Brust der Patientin ragt durch eine Öffnung in der Liege in den Strahlengang der Röntgenvorrichtung. Um nun während der Untersuchung konstante Verhältnisse zu erzeugen, wird die zu untersuchende Brust mit einem Stempel nach oben gedrückt und in eine vordefinierte Form gebracht. Durch eine Verschiebung des Stempels ist eine Anpassung an unterschiedliche Brustgrößen möglich. Allerdings ist durch die Kompression der Brust eine brustwandnahe Untersuchung nicht möglich. Eine andere Vorrichtung zur Stabilisierung der Brust der Patientin ist in der US 6,418,188 B1 offenbart. Ein Becher aus gummiartigem Gewebe wird über die Brust gestülpt und mittels einer Schnur von der Patientin weggezogen. Dadurch wird die Brust im Durchmesser komprimiert und in die Länge gezogen. Mit dieser Vorrichtung ist keine exakt reproduzierbare Lage und Form der Brust herstellbar. Durch Vakuumfixierungssysteme wie in der EP 1864611 A1 offenbart, wird die Brust durch Unterdruck in eine Schale gezogen. Dieser Unterdruck kann die Flüssigkeitsverteilung im Gewebe beeinflussen. Auch hier ist bei harten Schalen eine invasive diagnostische oder therapeutische Maßnahme wie Gewebeentnahme nicht möglich. Die Größe der Schale muss an die Größe der Brust angepasst werden, was die Handhabbarkeit erschwert.

### Darstellung der Erfindung

Der Erfindung liegt die Aufgabe zu Grunde, die Brust einer Patientin während einer diagnostischen Untersuchung und/oder einem therapeutischen Eingriff in einer reproduzierbaren Position zu fixieren und möglichst weit vom Brustkorb zu lösen, ohne die Lage und Darstellung der Gewebestrukturen der Brust negativ zu beeinflussen.

Diese Aufgabe wird durch eine Vorrichtung nach Anspruch 1 gelöst. Vorteilhafte Ausgestaltungen der Erfindung sind in den Unteransprüchen angegeben.

Eine erfindungsgemäße Vorrichtung zur Fixierung einer weiblichen Brust 31 einer Patientin 30 in einem medizinischen Untersuchungsgerät umfasst wenigstens eine elastische ringförmige Fixierung 110, 114. Diese umschließt die Brust an ihrem Ansatz an der Brustwand eng. Weiterhin wird durch diese Fixierung eine Kraft in einer Richtung von der Patientin weg ausgeübt. Dadurch kann die Brust geringfügig von der Brustwand abgezogen werden. Somit ist es einfacher möglich, den gesamten Bereich der Brust beispielsweise mit einem Röntgengerät zu erfassen.

In einer besonders vorteilhaften Ausgestaltung der Erfindung ist ein Netz 113 vorgesehen, welches an der elastischen ringförmigen Fixierung 110, 114 befestigt ist. Auch dieses Netz umschließt die übrige Brust eng. Hier wird nun eine Kraft auf das Netz in Richtung von der Patientin weg ausgeübt. Somit ergibt sich ein Zug auf das Netz selbst und auf die damit verbundene elastische ringförmige Fixierung.

Es vorteilhaft wenn die elastische ringförmige Fixierung 110, 114 mehrere Fixierschlingen-Zugschnüre umfasst. Diese können einzelne eingestellt werden und sind vorzugsweise in einem Winkel von 90 Grad zueinander angeordnet. Dadurch können jeweils paarweise gegeneinander angeordnete Fixierschlingen-Zugschnüre gegeneinander verspannt werden.

Vorteilhafterweise ist wenigstens ein Motor vorgesehen, welche die elastische ringförmige Fixierung 110, 114 zusammenzieht.

Weiterhin ist es vorteilhaft, wenn wenigstens ein Motor vorgesehen ist, der die elastische ringförmige Fixierung 110, 114 beziehungsweise das Netz 113 von der Patientin weg zieht.

Besonders vorteilhaft ist es, wenn der Motor mit einer Kraftmess- und Steuereinrichtung verbunden ist, welche den Motor beim Erreichen einer vorgegebenen Kraft abschaltet. Alternativ hierzu könnte der Motor über eine Regelschleife derart geregelt werden, dass er eine konstante Kraft ausübt.

Ein weiterer Aspekt der Erfindung betrifft ein Röntgengerät, insbesondere einen CT Scanner mit einer Fixiervorrichtung wie oben beschrieben.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt eine erfindungsgemäße Vorrichtung
Figur 2 zeigt eine besonders flexibel einstellbare erfindungsgemäße Vorrichtung
Figur 3 zeigt eine Draufsicht auf die Brust-Fixiervorrichtung.
Figur 4 zeigt ein Detail der Draufsicht auf die Brust-Fixiervorrichtung.
Figur 5 zeigt eine weitere Variante der Erfindung

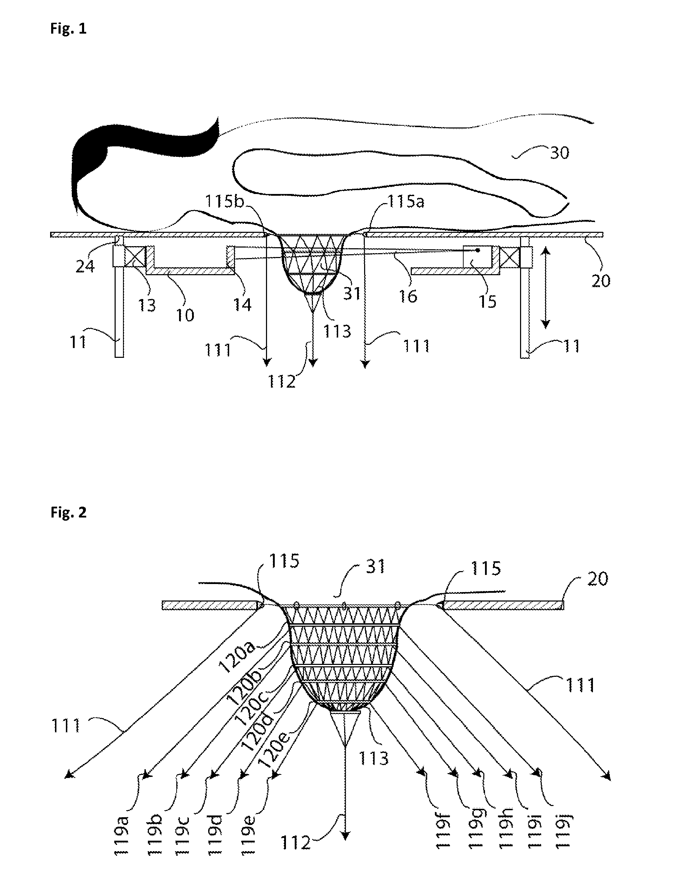

In Figur 1 ist eine erfindungsgemäße Vorrichtung in einem Röntgengerät im seitlichen Schnitt dargestellt. Die Patientin 30 ist auf der Patientenliege 20 gelagert. Unterhalb der Patientin befindet sich eine Spiral-Computertomographen Gantry 10. Die Brust 31 der Patientin hängt durch den Brustausschnitt 21 in den Aufnahmebereich dieser Gantry. Die Gantry 10 hat innerhalb des Gantrygehäuses 29 eine Röntgenröhre 15, welche den Strahlenfächer 16 erzeugt. Die Strahlung dieses Strahlenfächers durchdringt die Brust 31 und wird von dem Detektor 14 aufgefangen. Um die gesamte Brust abzubilden, lässt die Gantry sich durch das Gantrydrehlager 13 drehen. Gleichzeitig mit der Drehung wird über den Gantryhubantrieb 11 eine Verschiebung in Richtung der Patientin, hier in vertikaler Richtung, durchgeführt, so dass die Brust spiralförmig abgetastet wird. Erfindungsgemäß wird die Brust in einer Fixiervorrichtung fixiert, welche wenigstens eine Fixierschlinge 114 sowie ein Netz 113 umfasst. Mittels der Fixierschlingen-Zugschnüre 111 kann die wenigstens eine Fixierschlinge 114 verkleinert werden.

Weiterhin kann das Netz 113 durch die Netz-Zugschnur 112 von der Patientin weggezogen werden.

In Figur 2 ist eine besonders flexibel einstellbare Vorrichtung entsprechend der Erfindung dargestellt. Hier sind zusätzliche Spannschnüre 119a bis 119j vorgesehen, welche jeweils am Netz befestigt sind und die Brust an ihrem Umfang umfassen. Dadurch werden Zugringe 120a -120e gebildet. Durch Zug an einer Spannschnur in Pfeilrichtung kann hier der Umfang des entsprechenden Zugringes und somit auch des Netzes verringert und somit der Druck auf die Brust erhöht werden. So kann beispielsweise durch gleichzeitigen Zug auf die Schnüre 119a und 119j der Umfang des Netzes am Zugring 120a nahe an der Patientin verringert werden. Eine Verringerung des Umfangs des Zugringes 120e in der Nähe der Brustwarze erlauben die Spannschnüre 119e und 119f. Der Umfang der dazwischen liegenden Zugringe 120b, 120c, 120d mit den anderen hier dargestellten Spannschnüren 119b, 119i; 119c, 119h; 119d, 119g verändern. In dieser Figur sind beispielhaft 5 Paare von Spannschnüren dargestellt. Grundsätzlich ist aber jede andere Anzahl von Spannschnüren realisierbar. Ebenso ist es möglich, für einen Spannring anstatt einem Paar von Spannschnüren jede andere Anzahl von Spannschnüren, beispielsweise eine Spannschnur oder auch vier Spannschnüre einzusetzen.

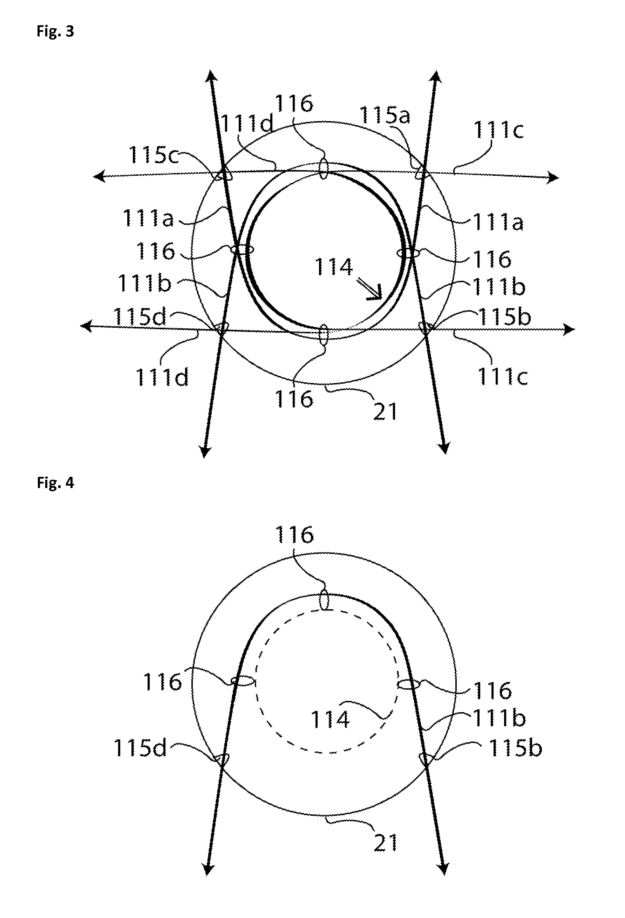

Figur 3 zeigt eine Draufsicht auf die Brust-Fixiervorrichtung, wie sie in Figur 1 und in Figur 2 dargestellt ist. Innerhalb des Brustausschnitts 21 der Patientenliege 20 sind Umlenkösen 115a, 115b, 115c, 115d angebracht. Diese führen die Fixierschlingen-Zugschnüre 111a, 111b, 111c, 111d. Die Fixierschlingen-Zugschnüre bestehen vorzugsweise aus einem elastischen Material, welches wiederum so steif ist, dass jedes auch im spannungsfreien Zustand die ungefähre Form der Schnüre wie dargestellt beibehält. Weiterhin sind noch Ringe 116 vorgesehen, welche die verschiedenen Fixierschlingen Zugschnüre zusammenhalten. Durch diese Fixierschlingen-Zugschnüre wird näherungsweise ein Kreis 114 gebildet.

Zur Fixierung werden in einem ersten Schritt die Fixierschlingen-Zugschnüre in Pfeilrichtung angezogen, so dass sich der Durchmesser des Kreises 114 an die Größe der Brust anpasst und diese eng umschließt. In einem zweiten Schritt wird dann das an den Fixierschlingen-Zugschnüren in Form eines Kreises befestigte Netz von der Patientin weggezogen. Anstelle der hier dargestellten vier Fixierschlingen-Zugschnüre ist auch jede andere Zahl von Fixierschlingen-Zugschnüren möglich. Grundsätzlich kann die Erfindung auch mit einer einzigen Fixierschlingen-Zugschnüre realisiert werden hierzu muss sie die gesamte Brust umschließen. Günstiger ist es, zwei Fixierschlingen-Zugschnüre, welche auf gegenüberliegenden Seiten der Brust angeordnet sind, zu verwenden. Die hier dargestellte Vorrichtung mit vier Fixierschlingen-Zugschnüren ermöglicht eine besonders gute Fixierung und einem gleichmäßigen Andruck der Schnüre an die Brust.

Figur 4 zeigt ein Detail der Draufsicht auf die Brust-Fixiervorrichtung. Es ist hier nur eine Fixierschlingen-Zugschnur 111b dargestellt. Die anderen Fixierschlingen-Zugschnüre wurden der Übersichtlichkeit halber weggelassen. Die Fixierschlingen-Zugschnur 111b wird durch die Ringe 116 um die Brust geführt. Durch die Ringe 116 werden auch andere Fixierschlingen-Zugschnüre geführt, so dass diese sich gegenseitig stabilisieren. Anstelle der Ringe 116 können auch beliebige andere Verbindungsteile eingesetzt werden, welche die einzelnen Schnüre lose aneinander zusammenhalten. Grundsätzlich ist es auch möglich, an diesen Ringen wieder weitere Zugschnüre zu befestigen, welche diese Ringe von der Brust weg ziehen. Dadurch kann eine Vorspannung auf die Fixierschlingen-Zugschnüre ausgeübt werden. Weiterhin kann damit die ganze Vorrichtung auf einfache Weise wieder geöffnet werden, sobald die Zugkraft von den Fixierschlingen-Zugschnüren nicht mehr besteht. Um eine automatische Öffnung zu erreichen, könnten auch Zugfedern an diesen Ringen angreifen, so dass diese nach außen, von der Brust weg gezogen werden. Um eine saubere Führung der Fixierschlingen-Zugschnüre zu erreichen, sind noch weitere Umlenkösen 115b und 115d vorgesehen. Diese ermöglichen beispielsweise eine Umlenkung der Fixierschlingen-Zugschnüre von der Patientin weg und aus dem Arbeitsbereich der Gantry. Eine solche Umlenkung nach unten ist in der Figur 1 dargestellt.

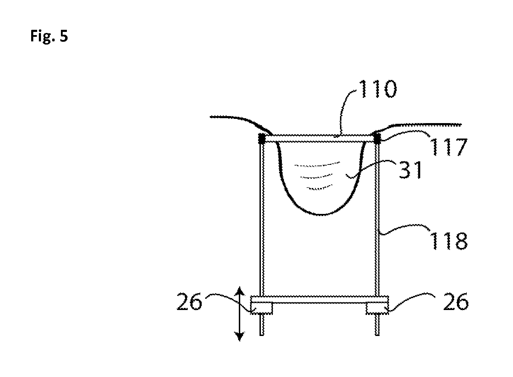

Figur 5 zeigt eine weitere Variante der Erfindung. Hierbei wird die Brust 31 durch einen elastischen Fixierungsring 110 umschlossen. Dieser Fixierungsring umfasst beispielsweise ein elastisches Kunststoffmaterial oder auch ein Gummimaterial. Der elastische Fixierungsring 110 ist an einer Positioniereinheit, hier umfassend die beiden Führungsstangen 118 sowie dem Hubantrieb 26 befestigt. Weiterhin ist wenigstens ein Kraftmesser 117 vorgesehen, welcher die Spannung des elastischen Fixierungsring oder die auf diesen ausgeübte Kraft überwacht, so dass auf die Brust keine zu große Kraft ausgeübt wird. Zur Fixierung der Brust wird in einem ersten Schritt der elastische Fixierungsring 110 zusammengezogen, so dass er sich dem Durchmesser der Brust anpasst und diese fest umschließt. Dann wird in einem zweiten Schritt dieser elastische Fixierungsring 110 von der Patientin mittels der Positioniereinheit weggezogen. Auch hier findet bevorzugt eine Überwachung der Kraft statt. Grundsätzlich ist möglich, das zusammenziehen, beziehungsweise das wegziehen von der Patientin so lange durchzuführen, bis eine maximale Kraft erreicht ist. Eine andere Alternative wäre eine konstante Kraftregelung, bei der beispielsweise der Hubantrieb 26 permanent eine konstante Kraft auf die Brust ausübt. Bei dieser Ausführungsform kann auch ein Netz über die Brust gezogen werden, ähnlich wie dies bei der ersten Ausführungsform der Erfindung beschrieben ist. In diesem Falle wird dann vorteilhafterweise das Netz selbst in Richtung von der Patientin weggezogen.

### Bezugszeichenliste

- 10: Gantry
- 11: Gantryhubantrieb
- 13: Gantrydrehlager
- 14: Detektor
- 15: Röntgenröhre
- 16: Strahlenfächer
- 20: Patientenliege
- 21: Brustausschnitt
- 24: Gantryaufhängung
- 26: Hubantrieb
- 30: Patientin
- 31: Brust
- 110: elastischer Fixierungsring
- 111: Fixierschlinge Zugschnur
- 112: Netz-Zugschnur
- 113: Netz
- 114: Fixierschlingen - Kreis
- 115: Umlenköse
- 116: Ring
- 117: Kraftmesser
- 118: Führungsstange
- 119: Spannschnur
- 120: Zugring

## Patentansprüche

1. Vorrichtung zur Fixierung einer weiblichen Brust (31) einer Patientin (30) in einem medizinischen Untersuchungsgerät umfassend eine elastische ringförmige Fixierung (110, 114), welche die Brust an ihrem Ansatz an der Brustwand eng umschließt und auf die eine Kraft in einer Richtung von der Patientin weg ausgeübt wird.

2. Vorrichtung nach Anspruch 1,
**dadurch gekennzeichnet, dass**
ein Netz (113) vorgesehen ist, welches an der elastischen ringförmigen Fixierung (110, 114) befestigt ist, und die übrige Brust eng umschließt, wobei eine Kraft auf das Netz in Richtung von der Patientin weg ausgeübt wird, um die Brust zu komprimieren und von der Brustwand abzuziehen.

3. Vorrichtung nach Anspruch 1 oder 2,
**dadurch gekennzeichnet, dass**
die elastische ringförmige Fixierung (110, 114) mehrere Fixierschlingen-Zugschnüre (111a, 111b, 111c, 111d) umfasst.

4. Vorrichtung nach einem der vorhergehenden Ansprüche,
**dadurch gekennzeichnet, dass**
wenigstens ein Motor vorgesehen ist, welcher die elastische ringförmige Fixierung (110, 114) zusammenzieht.

5. Vorrichtung nach einem der vorhergehenden Ansprüche,
**dadurch gekennzeichnet, dass**
wenigstens ein Motor vorgesehen ist, welcher die elastische ringförmige Fixierung (110, 114) und/oder das Netz (113) von der Patientin weg zieht.

6. Vorrichtung nach Anspruch 4 oder 5,
**dadurch gekennzeichnet, dass**
der wenigstens eine Motor mit einer Kraftmess- und Steuereinrichtung verbunden ist, welche den Motor beim Erreichen einer vorgegebenen Kraft abschaltet oder mit einer Regelschleife über den Motor eine konstante Kraft ausübt.

7. Röntgengerät, insbesondere CT-Scanner mit einer Vorrichtung zur Fixierung einer weiblichen Brust (31) einer Patientin (30) entsprechend einem der vorhergehenden Ansprüche.
